# EUROPÄISCHE PATENTSCHRIFT

(11) **EP 1 235 850 B1**
(45) Veröffentlichungstag und Bekanntmachung des Hinweises auf die Patenterteilung: **08.09.2004**
(21) Anmeldenummer: 00979607.9
(22) Anmeldetag: 21.11.2000
(51) Int. Cl.: C07J 53/00, A61K 31/565, A61P 5/26, A61P 5/34

(54) **UNGESÄTTIGTE 14,15-CYCLOPROPANO-ANDROSTANE, VERFAHREN ZU IHRER HERSTELLUNG UND DIESE VERBINDUNGEN ENTHALTENDE PHARMAZEUTISCHE ZUSAMMENSETZUNGEN**
UNSATURATED 14,15-CYCLOPROPANE-ANDROSTANES, A METHOD FOR THEIR PRODUCTION AND PHARMACEUTICAL COMPOSITIONS CONTAINING THESE COMPOUNDS
14,15-CYCLOPROPANO-ANDROSTANES INSATURES, LEUR PROCEDE DE PRODUCTION ET COMPOSITIONS PHARMACEUTIQUES CONTENANT CES COMPOSES

(30) Priorität: 08.12.1999 DE 19959696
(43) Veröffentlichungstag der Anmeldung: 04.09.2002
(73) Patentinhaber: Schering Aktiengesellschaft, 13353 Berlin (DE)
(72) Erfinder: RING, Sven, 07749 Jena (DE); ELGER, Walter, 14195 Berlin (DE); KAUFMANN, Günter, 07743 Jena (DE)
(74) Vertreter: Ziebig, Marlene, Dr. Dipl.-Chem.
(86) Internationale Anmeldenummer: PCT/EP2000/011557
(87) Internationale Veröffentlichungsnummer: WO 2001/042275

(56) Entgegenhaltungen:
- EP-A- 0 768 316
- WO-A-99/67275
- CH-A- 376 097
- DD-A- 31 269
- DD-A- 33 136
- GB-A- 839 908
- GB-A- 855 800
- GB-A- 874 572
- GB-A- 928 714
- US-A- 2 895 969
- US-A- 3 201 427
- US-A- 3 239 512
- BLACKMORE PETER F ET AL: "Unusual steroid specificity of the cell surface progesterone receptor on human sperm." MOLECULAR PHARMACOLOGY, Bd. 49, Nr. 4, 1996, Seiten 727-739, XP000992523 ISSN: 0026-895X

## Beschreibung

Die Erfindung betrifft neue ungesättigte 14,15-Cyclopropano-Androstane, deren Herstellung und diese Verbindungen enthaltende pharmazeutische Zusammensetzungen.

Ungesättigte 14,15-Cyclopropano-Androstane der folgenden Formel 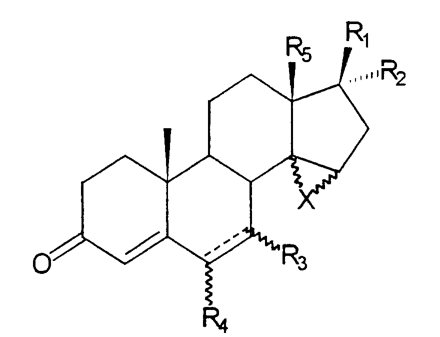 sind in der deutschen Anmeldung Nr. 198 27 523.4 (PCT/DE99/01794) beschrieben, die einen früheren Zeitrang als die vorliegende Anmeldung beansprucht, aber nach deren Einreichung veröffentlicht wird.

In der Formel ist R₁ ein Wasserstoffatom, eine Hydroxygruppe, eine Alkyloxy-, Acyloxy-, Aryloxy-, Aralkyloxy- oder eine Alkylaryloxygruppe, ein Rest -OCONHR₉ oder -OCOOR₉ mit R₉ für ein Wasserstoffatom, einen Alkyl-, Aryl- , Aralkyl- oder Alkylarylrest mit jeweils 1-10 Kohlenstoffatomen,
steht R₂ für ein Wasserstoffatom, eine Hydroxylgruppe eine Alkyl-, Acyl-, Aryl-, Aralkyl-, Alkylarylgruppe mit jeweils 1-10 Kohlenstoffatomen,
für einen Rest -(CH₂)ₙCH₂Y mit n = 0, 1 oder 2 und Y für ein Fluor-, Chlor-, Brom-, oder Jodatom, für eine Cyan-, Azid- oder Rhodanidgruppe, für einen Rest -OR₁₀ oder -SR₁₀ mit R₁₀ für ein Wasserstoffatom, einen Alkyl-, Aryl-, Aralkyl- oder Alkylarylrest mit jeweils 1-10 Kohlenstoffatomen oder einen Acylrest COR₉ mit R₉ für einen Alkyl-, Aryl-, Aralkyl- oder Alkylarylrest mit jeweils 1-10 Kohlenstoffatomen, einen Rest -OR₉ mit R₉ für ein Wasserstoffatom, einen Alkyl-, Aryl-, Aralkyl- oder Alkylarylrest mit jeweils 1-10 Kohlenstoffatomen,
für einen Rest -(CH₂)ₘ-CH=CH(CH₂)ₙ-R₈ mit m = 0, 1, 2 oder 3 und n = 0, 1 oder 2 und R₈ für ein Wasserstoffatom, einen Alkyl-, Aryl-, Aralkyl- oder Alkylarylrest mit jeweils 1-10 Kohlenstoffatomen oder eine Hydroxylgruppe, eine Alkoxygruppe oder Acyloxygruppe mit jeweils 1-10 Kohlenstoffatomen,
einen Rest -(CH₂)ₒC≡CR₁₁ mit o = 0,1 oder 2 und R₁₁ für ein Wasserstoffatom, ein Fluor-, Chlor-, Brom- oder lodatom, einen Alkyl-, Aryl-, Aralkyl-, Alkylaryl- oder einen Acylrest mit jeweils 1-10 Kohlenstoffatomen,
stehen R₁ und R₂ unabhängig voneinander für eine Keto-, Methylen-, Difluormethylengruppe,
kann sich zwischen C-6 und C-7 eine Doppelbindung befinden,
befindet sich zwischen C-14 und C-15 eine α- oder β- Cyclopropangruppe X mit X für eine CZ₂- Gruppe mit Z für ein Wasserstoff-, Fluor-, Chlor-, Brom- oder lodatom,
stehen R₃ und R₄ unabhängig voneinander für ein Wasserstoffatom, eine α- oder βständige Alkylgruppe mit 1-10 Kohlenstoffatomen und
steht R₅ für eine Alkylgruppe mit 1-3 Kohlenstoffatomen.

Aus EP 0 768 316 A1 sind Steroide mit einer 14,15-Methylengruppe bekannt, die progesterone Wirkung aufweisen und damit in Kombination mit mindestens einem geeigneten Estrogen zur hormonalen Kontrazeption und in der klimakterischen Hormon-Replacement-Therapie (HRT) sowie zur Behandlung der Endometriose oder gestagenabhängiger Tumore geeignet sind.

Gegenüber diesem Stand der Technik liegt der vorliegenden Erfindung die Aufgabe zugrunde, neue ungesättigte 14,15-Cyclopropano-Androstane, bereitzustellen.

Diese Aufgabe wurde durch ungesättigte 14,15-Cyclopropano-Androstane der allgemeinen Formel (I) 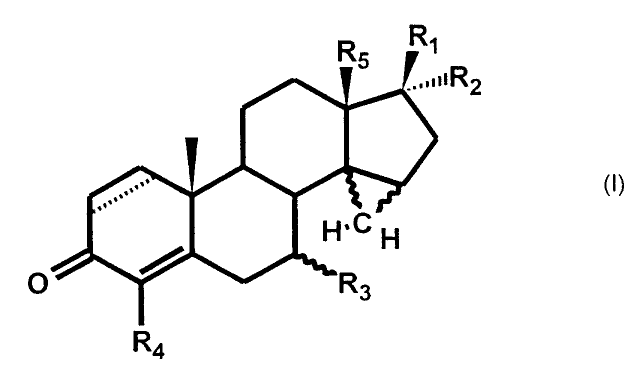 worin
R₁ ein Wasserstoffatom, eine Hydroxygruppe, eine C₁₋₁₀-Alkyl-, C₁₋₁₀-Alkyloxy-, C₁-₁₅-Acyloxy-, C₄₋₁₅-Aryloxy-, C₇₋₁₅-Aralkyloxy- oder eine C₇₋₁₅-Alkylaryloxygruppe ist,
R₂ für ein Wasserstoffatom, eine Hydroxygruppe, eine C₁₋₁₀-Alkyl-, C₁₋₁₀-Acyl-, C₁₋₁₀-Acyloxy-, C₆₋₁₅-Aryl-, C₇₋₁₅-Aralkyl- oder C₇₋₁₅-Alkylarylgruppe,
   für einen Rest -(CH₂)ₙCH₂Y
   mit n = 0, 1 oder 2 und Y für ein Halogenatom, insbesondere ein Fluor-, Chlor-, Brom- oder Jodatom, für ein Pseudohalogen, insbesondere eine Cyan-, Azid- oder Rhodanidgruppe,
   für einen Rest - (CH₂)m-CH=CH(CH₂)p-R₆
   mit m = 0, 1, 2 oder 3 und p = 0, 1 oder 2 und R₆ für ein Wasserstoffatom, einen C₁₋₁₀-Alkyl-, C₆₋₁₅-Aryl-, C₇₋₁₅-Aralkyl- oder C₇₋₁₅-Alkylarylrest oder eine Hydroxylgruppe, eine C₁₋₁₀-Alkyloxygruppe oder eine C₁₋₁₀-Acyloxygruppe,
   für einen Rest -(CH₂)oC≡CR₇
   mit o = 0, 1 oder 2 und R₇ für ein Wasserstoffatom, ein Halogenatom, insbesondere ein Fluor-, Chlor-, Brom- oder lodatom, einen C₁₋₁₀-Alkyl-, C₆₋₁₅-Aryl-, C₇₋₁₅-Aralkyl-, C₇₋₁₅-Alkylaryl- oder C₁₋₁₀-Acylrest steht,
   R₁ und R₂ zusammen für eine Keto-, Methylen-, Difluormethylengruppe stehen oder unter Einschluß des C-17 ein Spirooxiran oder ein 2,2-Dimethyl-1,3-dioxolan bilden,
   sich zwischen C-1 und C-2 eine Doppelbindung befinden kann,
   sich zwischen C-14 und C-15 eine α- oder β-Cyclopropangruppe befindet,
R₃ für ein Wasserstoffatom oder eine α- oder β-ständige C₁₋₁₀-Alkylgruppe steht,
R₄ für ein Halogenatom, insbesondere ein Fluor-, Chlor- oder Bromatom, oder ein Pseudohalogen, insbesondere eine Rhodanid- oder Azidgruppe, oder eine Hydroxyl- oder Perfluoralkylgruppe steht und
R₅ für eine C₁₋₄-Alkylgruppe steht,
mit der Maßgabe, daß, wenn sich in 1,2-Stellung eine Doppelbindung befindet, R₄ zusätzlich zu den voranstehenden Bedeutungen ein Wasserstoffatom sein kann, sowie deren pharmazeutisch verträgliche Salze, gelöst.

Es wurde überraschenderweise gefunden, daß die erfindungsgemäßen, ungesättigten 14,15-Cyclopropano-Androstane der allgemeinen Formel (I) gestagen und/oder androgen wirkende Verbindungen sind.

Im Sinne der Erfindung sind pharmazeutisch verträgliche Salze Alkali- oder Erdalkalisalze, insbesondere Natrium-, Kalium- oder Ammoniumsalze. Diese Salze können nach im Stand der Technik wohlbekannten Standardtechniken und -verfahren hergestellt werden.

Unter "C₁₋₄- bzw. C₁₋₁₀-Alkylgruppe" wird im Sinne der vorliegenden Erfindung ein verzweigter oder geradkettiger Alkylrest mit 1 bis 4 bzw. 1 bis 10 Kohlenstoffatomen verstanden. Als Beispiele seien eine Methyl-, Ethyl-, n-Propyl-, i-Propyl-, n-Butyl-, i-Butyl- oder tert.-Butyl-, n-Pentyl-, i-Pentyl-, n-Hexyl-, 2-Methylpentyl-, 3-Methylpentyl-, 2,2-Dimethylbutyl- oder 2,3-Dimethylbutylgruppe genannt.

Unter dem Begriff "C₁₋₁₀-Alkyloxygruppe" wird im Sinne der vorliegenden Anmeldung eine cyclische oder acyclische Gruppe, deren Alkylrest 1 bis 10 Kohlenstoffatome aufweist, verstanden, wobei unter einer "cyclischen Gruppe" auch eine heterocyclische Gruppe verstanden wird, die im Ring ein oder zwei Heteroatome aufweisen kann, die aus einem Stickstoffatom, einem Sauerstoffatom und einem Schwefelatom ausgewählt werden können. Beispiele sind eine Methoxygruppe, Ethoxygruppe oder n- oder iso-Propoxygruppe, iso- oder tert.-Butoxygruppe, eine (1'-Methoxy)cyclopentoxy- bzw. Tetrahydropyranyloxygruppe.

Unter dem Begriff "C₁₋₁₀- bzw. C₁₋₁₅-Acyl- bzw. Acyloxygruppe" wird im Sinne der vorliegenden Anmeldung ein Rest mit 1 bis 10 bzw. 1 bis 15 Kohlenstoffatomen der geradkettigen oder verzweigten Alkancarbonsäuren, wie beispielsweise der Ameisensäure, Essigsäure, Propionsäure, Butansäure, iso-Butansäure, Heptansäure oder Undecansäure, verstanden.

Unter dem Begriff "C₆₋₁₅-Arylgruppe" wird im Sinne der vorliegenden Anmeldung ein substituierter oder unsubstituierter Arylrest mit 6 bis 15 Kohlenstoffatomen, beispielsweise eine Phenylgruppe, eine substituierte Phenylgruppe, wie eine Halogenphenylgruppe oder eine Nitrophenylgruppe, oder eine Naphtylgruppe verstanden.

Unter dem Begriff "C₄₋₁₅-Aryloxygruppe" wird im Sinne der vorliegenden Anmeldung ein carbocyclischer oder heterocyclischer Rest mit 4 bis 15 Kohlenstoffatomen verstanden. Beispiele sind eine Benzoyloxygruppe, 1- oder 2-Naphtinyloxygruppe, 2- oder 3-Furanyloxygruppe, 2- oder 3-Thienyloxygruppe, 2-,3- oder 4-Pyridinyloxygruppe.

Unter dem Begriff "C₇₋₁₅-Alkylarylgruppe" wird im Sinne der vorliegenden Anmeldung ein mit einem Alkylrest substituierter Arylrest, die zusammen 7 bis 15 Kohlenstoffatomen aufweisen, verstanden, wobei der Arylrest weitere Substituenten, wie beispielsweise ein Halogenatom tragen kann. Beispiele sind eine Toluenylgruppe (Methylphenylgruppe), Halogentoluenylgruppe, Ethylphenylgruppe, Dimethylphenylgruppe oder Trimethylphenylgruppe.

Unter dem Begriff "C₇₋₁₅-Alkylaryloxygruppe" wird im Sinne der vorliegenden Anmeldung eine um ein Sauerstoffatom verlängerte "C₇₋₁₅-Aralkylgruppe", wie beispielsweise eine 3-bzw. 4-Methylphenyloxygruppe, verstanden.

Unter dem Begriff "C₇₋₁₅-Aralkylgruppe" wird im Sinne der vorliegenden Anmeldung ein mit einem Arylrest substituierter Alkylrest, die zusammen 7 bis 15 Kohlenstoffatomen aufweisen, verstanden, wobei der Arylrest weitere Substituenten, wie beispielsweise ein Halogenatom tragen kann. Beispiele sind eine freie oder aromatisch substituierte Benzylgruppe, wie eine Benzylgruppe oder eine Halogenbenzylgruppe.

Unter dem Begriff "C₇₋₁₅-Aralkyloxygruppe" wird im Sinne der vorliegenden Anmeldung eine um ein Sauerstoffatom verlängerte "C₇₋₁₅-Aralkylgruppe", wie beispielsweise eine Benzyloxygruppe, verstanden.

Unter dem Begriff "Halogen" wird im Rahmen der vorliegenden Erfindung ein Fluor-, Chlor-, Brom- oder lodatom verstanden.

Unter dem Begriff "Pseudohalogen" wird im Sinne der vorliegenden Anmeldung insbesondere eine Cyanat-, Rhodanid-, Cyan- oder Azidgruppe verstanden.

Unter dem Begriff "Perfluoralkylrest" wird im Sinne der vorliegenden Anmeldung ein verzweigter oder geradkettiger Fluoralkylrest mit 1 bis 3 Kohlenstoffatomen verstanden, wobei Beispiele eine Trifluormethyl-, Pentafluorethyl-, Heptafluor-n-propyl- oder Heptafluor-iso-propylgruppe sind.

R₁ bedeutet vorzugsweise eine Hydroxy- oder Acyloxygruppe, insbesondere eine Hydroxygruppe, Formyloxygruppe, Acetyloxygruppe, Propionyloxygruppe, n-Butyryloxygruppe, iso-Butyryloxygruppe, Heptanyloxygruppe oder Undecanyloxygruppe.

Bedeutet R₂ einen Rest -(CH₂)ₙCH₂Y ist n vorzugsweise gleich 1 und Y steht vorzugsweise für ein Fluoratom, eine Cyan- oder Rhodanidgruppe. Bedeutet R₂ einen Rest - (CH₂)ₘ-CH=CH(CH₂)ₚ-R₆ ist m vorzugsweise gleich 1 und R₆ steht vorzugsweise für eine Methyl- oder Ethylgruppe oder eine Methoxy- oder Ethoxygruppe.

Bedeutet R₂ einen Rest -(CH₂)ₒC≡CR₇ ist o vorzugsweise gleich 1 und R₇ steht vorzugsweise für ein Fluoratom oder für eine Methyl- oder Ethylgruppe.

Besonders bevorzugt bedeutet R₂ ein Wasserstoffatom oder eine C₁₋₆-Alkylgruppe, insbesondere eine Methyl- oder Ethylgruppe.

R₃ bedeutet vorzugsweise einen C₁₋₄-Alkylrest, besonders bevorzugt eine Methylgruppe.

R₄ bedeutet vorzugsweise ein Fluor-, Chlor- oder Bromatom oder eine Trifluormethyl- oder Hydroxygruppe.

R₅ bedeutet vorzugsweise eine Methyl- oder Ethylgruppe.

Am stärksten bevorzugt sind die folgenden Verbindungen:
1) 4-Chlor-17β-Hydroxy-14α, 15α-methylen-androst-4-en-3-on,
2) 4-Chlor-17α-Hydroxy-14α, 15α-methylen-androst-4-en-3-on,
3) 4-Chlor-17β-Hydroxy-14β, 15β-methylen-androst-4-en-3-on,
4) 4-Chlor-17α-Hydroxy-14β, 15β-methylen-androst-4-en-3-on,
5) 4-Brom-17β-Hydroxy-14α, 15α-methylen-androst-4-en-3-on,
6) 4-Brom-17α-Hydroxy-14α, 15α-methylen-androst-4-en-3-on,
7) 4-Brom-17β-Hydroxy-14β,15β-methylen-androst-4-en-3-on,
8) 4-Brom-17α-Hydroxy-14β,15β-methylen-androst-4-en-3-on,
9) 4-Fluor-17β-Hydroxy-14α, 15α-methylen-androst-4-en-3-on,
10) 4-Fluor-17α-Hydroxy-14α, 15α-methylen-androst-4-en-3-on,
11) 4-Fluor-17β-Hydroxy-14β,15β-methylen-androst-4-en-3-on,
12) 4-Fluor-17α-Hydroxy-14β, 15β-methylen-androst-4-en-3-on,
13) 4,17β-Dihydroxy-14α, 15α-methylen-androst-4-en-3-on,
14) 4,17α-Dihydroxy-14α, 15α-methylen-androst-4-en-3-on,
15) 4,17β-Dihydroxy-14β, 15β-methylen-androst-4-en-3-on,
16) 4,17α-Dihydroxy-14β, 15β-methylen-androst-4-en-3-on,
17) 4-Trifluormethyl-17β-Hydroxy-14α, 15α-methylen-androst-4-en-3-on,
18) 4-Trifluormethyl-17α-Hydroxy-14α, 15α-methylen-androst-4-en-3-on,
19) 4-Trifluormethyl-17β-Hydroxy-14β, 15β-methylen-androst-4-en-3-on,
20) 4-Trifluormethyl-17α-Hydroxy-14β, 15β-methylen-androst-4-en-3-on,
21) 17β-Hydroxy-14α, 15α-methylen-androsta-1,4-dien-3-on,
22) 17α-Hydroxy-14α, 15α-methylen-androsta-1,4-dien-3-on,
23) 17β-Hydroxy-14β, 15β-methylen-androsta-1,4-dien-3-on,
24) 17α-Hydroxy-14β, 15β-methylen-androsta-1,4-dien-3-on,
25) 4-Chlor-17α-Hydroxy-14α, 15α-methylen-androsta-1,4-dien-3-on,
26) 4-Chlor-17β-Hydroxy-14α, 15α-methylen-androsta-1,4-dien-3-on,
27) 4-Chlor-17β-Hydroxy-14β, 15β-methylen-androsta-1,4-dien-3-on und
28) 4-Chlor-17α-Hydroxy-14β, 15β-methylen-androsta-1,4-dien-3-on.

Die erfindungsgemäßen Verbindungen und deren pharmazeutisch annehmbare Salze können hergestellt werden, indem man in Verbindungen der allgemeinen Formel (II) 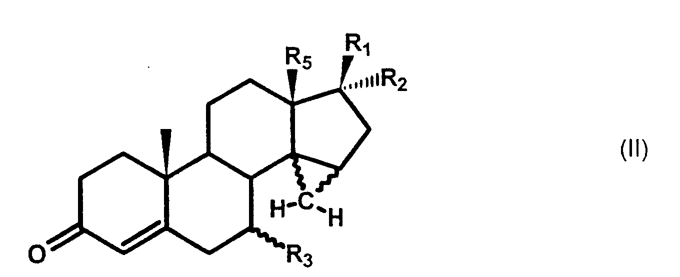 worin R₁, R₂, R₃ und R₅ die vorstehend gegebene Bedeutung besitzen, sich zwischen C-14 und C-15 eine α- oder β-Cyclopropangruppe befindet, die 4,5-Doppelbindung mit Wasserstoffperoxid unter alkalischen Bedingungen epoxidiert und das entstehende Epoxidgemisch in einem geeignetem Lösungsmittel mit Säuren der allgemeinen Formel HR₈ behandelt, wobei R₈ ein Halogenatom oder ein Pseudohalogen sein kann.

Des weiteren können entsprechende 4-Bromverbindungen auch durch Addition von Brom mittels Brom, N-Bromsuccinimid oder N-Bromacetamid an Verbindungen der allgemeinen Formel (II) in Essigsäure/Ether in Gegenwart eines Protonenakzeptors, wie zum Beispiel Kollidin, hergestellt werden (X. S. Fei et. al., J. Chem. Soc. Perkin Trans.1, 1998, 1139-1142).

4-Hydroxyverbindungen werden durch Umsetzung des oben genannten Epoxidgemisches mit katalytischen Mengen Mineralsäure, wie zum Beispiel Schwefelsäure, erhalten (P. S. Furth et. al. J. Enzyme Inhibition, 1990 , Vol.4, 131-135).

Verbindungen der allgemeinen Formel (I) mit einer zusätzlichen Doppelbindung in 1,2-Stellung können leicht nach den dem Fachmann bekannten Methoden, wie zum Beispiel Dehydrierung des 4-En-3-on-Systems mittels 2,3-Dichlor-5,6-dicyanbenzochinon in einem geeignetem Lösungsmittel, wie zum Beispiel Dioxan, Toluol oder tert.-Butanol erhalten werden.

4-Trifluormethylverbindungen der allgemeinen Formel (I) können durch Umsetzung der voranstehend genannten 4-Bromverbindungen der allgemeinen Formel (I) mit 2,2-Difluor-2-(fluorsulfonyl)essigsäuremethylester in Dimethylformamid in Gegenwart von CuJ erhalten werden (X. S. Fei et. al., J. Chem. Soc., Perkin Trans. 1, 1998, 1139-1142). Die Ausgangsverbindungen der Formel (II) können gemäß bekannter Verfahren bzw. nach den in der deutschen Anmeldung mit der Anmeldenummer 198 27 523.4 (PCT/DE99/01794) beschriebenen Verfahren hergestellt werden. In dem genannten Schutzrecht ist auch die Einführung der zu den hier beanspruchten Resten R₁, R₂, R₃ und R₅ dort analog vorkommenden Resten beschrieben.

Gegenstand der vorliegenden Erfindung sind auch pharmazeutische Zusammensetzungen zur oralen, rektalen, subcutanen, intravenösen oder intramuskulären Anwendung, die zusammen mit den üblichen Trägern und Verdünnungsmitteln mindestens eine Verbindung der allgemeinen Formel (I) und/oder deren Säureadditionssalze als Wirkstoff enthalten.

Pharmazeutische Präparate der Erfindung werden mit den üblichen festen oder flüssigen Trägerstoffen und/oder Verdünnungsmitteln und den allgemein üblicherweise verwendeten Hilfsstoffen entsprechend der gewünschten Applikationsart in einer geeigneten Dosierung und in an sich bekannter Weise hergestellt. Bei einer bevorzugten oralen Darreichungsform werden vorzugsweise Tabletten, Filmtabletten, Dragees, Kapseln, Pillen, Pulver, Lösungen oder Suspensionen auch als Depotform zubereitet.

Daneben sind parenterale Arzneiformen wie Injektionslösungen oder aber Suppositorien in Betracht zu ziehen.

Arzneiformen als Tabletten können beispielsweise durch Mischen des Wirkstoffes mit bekannten Hilfsstoffen, wie Dextrose, Zucker, Sorbit, Mannit, Polyvinylpyrrolidon, Sprengmitteln, wie Maisstärke oder Alginsäure, Bindemitteln, wie Stärke oder Gelatine, Gleitmitteln, wie Magnesiumstearat oder Talk, und/oder Mitteln, die einen Depoteffekt erzielen können, wie Carboxylpolymethylen, Carboxymethylcellulose, Celluloseacetatphthalat oder Polyvinylacetat, erhalten werden. Die Tabletten können auch aus mehreren Schichten bestehen.

Analog lassen sich Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker bereiten. Die Drageehülle kann dabei auch aus mehreren Schichten bestehen, wobei beispielsweise die oben genannten Hilfsstoffe verwendet werden.

Die Lösungen oder Suspensionen mit dem erfindungsgemäßen Wirkstoff können zur Verbesserung des Geschmacks mit Stoffen, wie Saccharin, Cyclamat oder Zucker und/oder mit Aromastoffen, wie Vanillin oder Orangenextrakt, versetzt werden. Weiterhin können sie mit Suspendierhilfsstoffen, wie Natriumcarboxymethylcellulose, oder Konservierungsmitteln, wie p-Hydroxybenzoesäure, vermischt werden.

Die Bereitung von Kapseln kann durch Mischen des Arzneistoffes mit Trägern, wie Milchzucker oder Sorbit, erfolgen, die dann in die Kapseln eingebracht werden.

Die Herstellung von Suppositorien erfolgt vorzugsweise durch Mischung des Wirkstoffes mit geeigneten Trägermaterialien, wie Neutralfetten oder Polyethylenglykolen, oder deren Derivaten.

Bei den pharmazeutischen Zubereitungsformen kann es sich weiterhin um perkutane Zubereitungsformen, z. B. transdermale, therapeutische Systeme (TTS) oder Gele, Sprays oder Salben oder um intranasale Zubereitungsformen, wie Nasenspray oder Nasentropfen, handeln.

Die erfindungsgemäßen, ungesättigten 14,15-Cyclopropano-Androstane der allgemeinen Formel (I) sind hormonell (gestagen und/oder androgen wirkende) Verbindungen.

So ist beispielsweise die Verbindung der allgemeinen Formel (I), in der R₁ eine Hydroxylgruppe, R₂ und R₃ ein Wasserstoffatom, R₅ eine Methylgruppe und X eine CH₂-Gruppe darstellen und der 14,15-Cyclopropanring α-ständig ist, nämlich 4-Chlor-17β-Hydroxy-14α, 15α-methylen-androst-4-en-3-on ein Androgen.

Während die Substanz 4-Chlor-17β-Hydroxy-14α, 15α-methylen-androst-4-en-3-on mit 42 ± 3 % an den Androgenrezeptor der Rattenprostata (Referenzsubstanz: 17β-Hydroxy-17α-methyl-estra-4,9,11-trien-3-on; R 1881) bindet, besteht praktisch keine Bindung an den Progesteronrezeptor des Kaninchenuterus (Referenzsubstanz: Progesteron). Im Hershberger-Test konnte eine deutliche androgene Aktivität nachgewiesen werden, demgegenüber ist die gestagene Wirkung im Graviditätserhaltungstest kaum vorhanden.

Diese Testergebnisse eröffnen den erfindungsgemäßen Verbindungen der allgemeinen Formel (I) vielfältige Möglichkeiten für die Fertilitätskontrolle beim Mann, die Hormon-Replacement-Therapie (HRT) bei Mann und Frau oder die Behandlung hormonell bedingter Erkrankungen bei Mann und Frau, wie beispielsweise Endometriose, Mammacarcinom oder Hypogonadismus.

Die nachfolgenden Beispielen dienen der näheren Erläuterung der vorliegenden Erfindung und sollen diese nicht einschränken.

### Beispiele

### Beispiel 1

### 17β-Hydroxy-4,5-epoxy-14α, 15α-methylen-androstan-3-on Herstellung der 4,5-Epoxide

2 g 17β-Hydroxy -14α, 15α-methylen-androst-4-en-3-on werden in 80 ml Methanol gelöst und bei 0°C mit 26 ml Wasserstoffperoxidlösung (35%) versetzt. Unter Rühren werden 5,2 ml 10 %-ige Natriumhydroxidlösung zugegeben und es wird bei 0°C 30 Stunden gerührt. Die Reaktionslösung wird mit 50 ml Dichlormethan und 25 ml Wasser versetzt und die organische Phase wird abgetrennt. Man wäscht mit halbkonzentrierter Thiosulfatlösung, trocknet und engt bis zur Trockene ein. Der erhaltene Rückstand besteht aus einem Gemisch von 4α,5α- bzw. 4β,5β-Epoxiden und wird ohne weitere Reinigung in der Folgestufe eingesetzt.

### Beispiel 2

### 17α-Hydroxy-4,5-epoxy-14α,15α-methylen-androstan-3-on

Die vorstehend genannte Verbindung kann in zu Beispiel 1 analoger Weise aus 17α-Hydroxy-14α,15α-methylen-androst-4-en-3-on gewonnen werden.

### Beispiel 3

### 4-Chlor-17β-hydroxy-14α,15α-methylen-androst-4-en-3-on

1,5 g 17β-Hydroxy-4,5-epoxy-14α,15α-methylen-androstan-3-on werden in 150 ml Aceton gelöst und bei 0°C mit 5,5 ml konzentrierter Salzsäure versetzt. Nach 24 Stunden bei 0°C wird mit Sodalösung neutralisiert und das Aceton wird abgezogen. Der Rückstand wird mit Dichlormethan ausextrahiert. Die organischen Extrakte werden getrocknet und eingeengt. Nach Kristallisation aus Ethanol erhält man 4-Chlor-17β-hydroxy-14α, 15α-methylen-androst-4-en-3-on.
¹H-NMR: 0,12 (1H, dd, J=5.5, 3.3 Hz, CH₂-Brücke), 0,22 (1H, dd, J=8.2, 5.5 Hz, CH₂-Brücke), 0,99 (3H, s, H-18), 1,30 (3H, s, H-19), 3,49 (1H, dd, J=9.3, 7.1 Hz, H-17).

### Beispiel 4

### 4-Chlor-17α-hydroxy-14α, 15α-methylen-androst-4-en-3-on

17α-Hydroxy-14α,15α-methylen-androst-4-en-3-on wird analog Beispiel 3 umgesetzt.
¹H-NMR: 0,32 (1H, dd, J=7.7, 4.9 Hz, CH₂-Brücke), 0,72 (1H, dd, J=4,4, 3,3 Hz, CH₂-Brücke), 0,99 (3H, s, H-18), 1,29 (3H, s, H-19), 3,80 (1H, d, J=6.0 Hz, H-17).

### Beispiel 5

### 4,17β-Dihydroxy-14α,15α-methylen-androst-4-en-3-on

3,5 g Epoxidgemisch, 17β-Hydroxy-4,5-epoxy-14α, 15α-methylen-androstan-3-on, (Stufe 1) werden in 50 ml Essigsäure, welche 2 Vol.-% konzentrierte Schwefelsäure enthält, gelöst. Die Lösung wird 3 Tage bei 10°C stehengelassen. Danach versetzt man mit 200 ml Essigsäureethylester und neutralisiert mit Sodalösung. Die organische Phase wird getrocknet und eingeengt. Der Rückstand wird in 100 ml Methanol gelöst, mit 4 g Kaliumhydroxid versetzt und 1 Stunde am Rückfluß erhitzt. Man läßt abkühlen, neutralisiert mit 50 %-iger Essigsäure, gießt in 1 1 Wasser und saugt die Kristalle ab. Man erhält 4,17β-Dihyxdroxy-14α, 15α-methylen-androst-4-en-3-on.
¹H-NMR: 0,13 (1H, dd, J=5,6, 3,2 Hz, CH₂-Brücke), 0,24 (1H, dd, J=8,3, 5,6 Hz, CH₂-Brücke), 0,99 (3H, s, H-18), 1,30 (3H, s, H-19), 3,50 (1H, dd, J=9.4, 6.8 Hz, H-17), 6.10 (1H,s, 4-OH).

### Beispiel 6

### 4-Brom-17β-hydroxy-14α, 15α-methylen-androst-4-en-3-on

Die Zielverbindung wird in für die Herstellung von 4-Chlor-17β-hydroxy-14α,15α-methylen-androst-4-en-3-on analoger Weise hergestellt, wobei anstatt Salzsäure 48 %ige Bromwasserstoffsäure verwendet wird.
¹H-NMR: 0,12 (1H, dd, J=5,5, 3,3 Hz, CH₂-Brücke), 0,21 (1H, dd, J=8,4, 5,4 Hz, CH₂-Brücke), 1,00 (3H, s, H-18), 1,33 (3H, s, H-19), 3,49 (1H, dd, J=9,3, 7,1 Hz, H-17).

### Beispiel 7

### 4-Trifluormethyl-17β-hydroxy-14α,15α-methylen-androst-4-en-3-on

1,5 g 4-Brom-17β-hydroxy-14α,15α-methylen-androst-4-en-3-on werden in 180 ml Dimethylformamid gelöst und mit 1 g Cul sowie 2,8 ml 2,2-Difluor-2-(fluorsulfonyl)-essigsäuremethylester bei 75 °C 12 Stunden gerührt. Nach Aufarbeitung und chromatographischer Reinigung erhält man 4-Trifluormethyl-17β-hydroxy-14α,15α-methylen-androst-4-en-3-on.
¹H-NMR: 0,14 (1H, dd, J=5,5, 3,0 Hz, CH₂-Brücke), 0,25 (1H, dd, J=8,2, 5,8 Hz, CH₂-Brücke), 1,00 (3H, s, H-18), 1,32 (3H, s, H-19), 3,51 (1 H, m, H-17).
¹⁹F-NMR: -55,3 (3F, s , 4-F₃C).

### Beispiel 8

### 17β-Hydroxy-14α,15α-methyten-androsta-1,4-dien-3-on

4 g 17β-Hydroxy-14α, 15α-methylen-androst-4-en-3-on werden in 160 ml Toluol mit 3,2 g 2,3-Dichlor-5,6-dicyanbenzochinon 6 Tage bei 85 °C gerührt. Es wird vom Niederschlag abgesaugt, mit wenig Toluol gewaschen und das Filtrat wird zur Trockene eingeengt.

Der Rückstand wird durch Chromatographie gereinigt und man erhält 17ß-Hydroxy-14α,15α-methylen-androsta-1,4-dien-3-on.
¹H-NMR: 0,13 (1H, dd, J=5,6, 3,2 Hz, CH₂-Brücke), 0,24 (1H, dd, J=8,3, 5,6 Hz, CH₂-Brücke), 0,98 (3H, s, H-18), 1,35 (3H, s, H-19), 3,50 (1H, m, H-17), 6,06 (1H, m., H-4); 6,22 (1 H, dd J=12,09;1,65 Hz, H{2}), 7,04 (1H, d, J=9,9 Hz, H-1).

### Beispiel 9

### 4-Chlor-17β-hydroxy -14α, 15α-methylen-androsta-1,4-dien-3-on

Wird in zu Beispiel 6 analoger Weise aus 4-Chlor-17β-hydroxy-14α,15α-methylen-androst-4-en-3-on hergestellt.
¹H-NMR: 0,13 (1H, dd, J=5,6, 3,2 Hz, CH₂-Brücke), 0,24 (1H, dd, J=8,3, 5,6 Hz, CH₂-Brücke), 0,98 (3H, s, H-18), 1,35 (3H, s, H-19), 3,50 (1H, m, H-17), 6,22 (1 H, dd J=12,09, 1.65 Hz, H{2}), 7,04 (1H, d, J=9,9 Hz, H-1).

## Patentansprüche

1. Ungesättigte 14,15-Cyclopropano-Androstane der allgemeinen Formel (I) 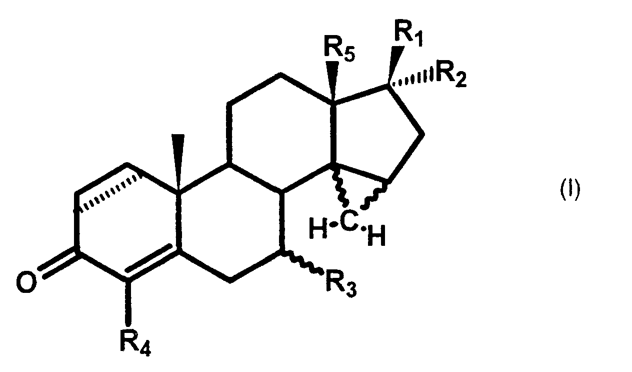 worin
R₁ ein Wasserstoffatom, eine Hydroxygruppe, eine C₁₋₁₀-Alkyl-, C₁₋₁₀-Alkyloxy-, C₁₋₁₅-Acyloxy-, C₄₋₁₅-Aryloxy-, C₇₋₁₅-Aralkyloxy- oder eine C₇₋₁₅-Alkylaryloxygruppe ist,
R₂ für ein Wasserstoffatom, eine Hydroxygruppe, eine C₁₋₁₀-Alkyl-, C₁₋₁₀-Acyl-, C₁₋₁₀-Acyloxy-, C₆₋₁₅-Aryl-, C₇₋₁₅-Aralkyl- oder C₇₋₁₅-Alkylarylgruppe,
für einen Rest -(CH₂)ₙCH₂Y
mit n = 0, 1 oder 2 und Y für ein Halogenatom, insbesondere ein Fluor-, Chlor-, Brom- oder Jodatom, für ein Pseudohalogen, insbesondere eine Cyan-, Azid- oder Rhodanidgruppe,
für einen Rest - (CH₂)m-CH=CH(CH₂)p-R₆
mit m = 0, 1, 2 oder 3 und p = 0, 1 oder 2 und R₆ für ein Wasserstoffatom, einen C₁. ₁₀-Alkyl-, C₆₋₁₅-Aryl-, C₇₋₁₅-Aralkyl- oder C₇₋₁₅-Alkylarylrest oder eine Hydroxylgruppe, eine C₁₋₁₀-Alkyloxygruppe oder eine C₁₋₁₀-Acyloxygruppe,
für einen Rest -(CH₂)oC≡CR₇
mit o = 0, 1 oder 2 und R₇ für ein Wasserstoffatom, ein Halogenatom, insbesondere ein Fluor-, Chlor-, Brom- oder lodatom, einen C₁₋₁₀-Alkyl-, C₆₋₁₅-Aryl-, C₇₋₁₅-Aralkyl-, C₇₋₁₅-Alkylaryl- oder C₁₋₁₀-Acylrest steht,
R₁ und R₂ zusammen für eine Keto-, Methylen-, Difluormethylengruppe stehen oder unter Einschluß des C-17 ein Spirooxiran oder ein 2,2-Dimethyl-1,3-dioxolan bilden,
sich zwischen C-1 und C-2 eine Doppelbindung befinden kann,
sich zwischen C-14 und C-15 eine α-oder β-Cyclopropangruppe befindet,
R₃ für ein Wasserstoffatom oder eine α- oder β-ständige C₁₋₁₀-Alkylgruppe steht,
R₄ für ein Halogenatom, insbesondere ein Fluor-, Chlor- oder Bromatom, oder ein Pseudohalogen, insbesondere eine Rhodanid- oder Azidgruppe, oder eine Hydroxyl- oder C₁₋₃-Perfluoralkylgruppe steht und
R₅ für eine C₁₋₄-Alkylgruppe steht,
worin C₁₋₁₀ Alkyloxygruppe eine heterocylische, cyclische oder acyclische Gruppe, deven Alkyl rest 1 bis 10 kohlenstoffatomen aufweist, darstellt, worin die heterocyclishe Gruppe 1 oder 2 heteroatomen in Ring aufweisen kann die aus N, O und S ausgewäht werden können,
worin die Arylrest der C₆₋₁₅-Aryl-, C₇₋₁₅-Alkylaryl-, C₇₋₁₅-Alkylaryloxy, C₇₋₁₅- Aralkyl-und C₇₋₁₅-Aralkyloxygruppe weiter substituiert sein können,
mit der Maßgabe, daß, wenn sich in 1,2-Stellung eine Doppelbindung befindet, R₄ zusätzlich zu den voranstehenden Bedeutungen ein Wasserstoffatom sein kann, sowie deren pharmazeutisch verträglichen Salze.

2. Verbindungen nach Anspruch 1, wobei R₁ eine Hydroxy- oder C₁₋₁₅-Acyloxyguppe, insbesondere eine Formyloxygruppe, Acetyloxygruppe, Propionyloxygruppe, Butyryloxygruppe, iso-Butyryloxygruppe, Heptanyloxygruppe oder Undecanyloxygruppe, darstellt.

3. Verbindungen nach Anspruch 1 oder 2, wobei R₂ ein Wasserstoffatom oder eine Alkylgruppe, insbesondere ein Methyl- oder Ethylgruppe, darstellt.

4. Verbindungen nach einem der Ansprüche 1 bis 3, wobei R₃ eine Methylgruppe ist.

5. Verbindungen nach einem der Ansprüche 1 bis 4, wobei R₄ ein Fluor-, Chlor- oder Bromatom oder eine Trifluormethyl- oder Hydroxygruppe darstellt.

6. Verbindungen nach einem der Ansprüche 1 bis 5, wobei R₅ eine Methyl- oder Ethylgruppe darstellt.

7. Verbindungen nach Anspruch 1, nämlich
1) 4-Chlor-17β-Hydroxy-14α, 15α-methylen-androst-en-3-on,
2) 4-Chlor-17α-Hydroxy-14α, 15α-methylen-androst-4-en-3-on,
3) 4-Chlor-17β-Hydroxy-14β, 15β-methylen-androst-4-en-3-on,
4) 4-Chlor-17α-Hydroxy-14β, 15β-methylen-androst-4-en-3-on,
5) 4-Brom-17β-Hydroxy-14α, 15α-methylen-androst-4-en-3-on,
6) 4-Brom-17α-Hydroxy-14α, 15α-methylen-androst-4-en-3-on,
7) 4-Brom-17ß-Hydroxy-14β, 15β-methylen-androst-4-en-3-on,
8) 4-Brom-17α-Hydroxy-14β, 15β-methylen-androst-4-en-3-on,
9) 4-Fluor-17β-Hydroxy-14α, 15α-methylen-androst-4-en-3-on,
10) 4-Fluor-17α-Hydroxy-14α, 15α-methylen-androst-4-en-3-on,
11) 4-Fluor-17β-Hydroxy-14β,15β-methylen-androst-4-en-3-on,
12) 4-Fluor-17α-Hydroxy-14β, 15β-methylen-androst-4-en-3-on,
13) 4,17β-Dihydroxy-14α, 15α-methylen-androst-4-en-3-on,
14) 4,17α-Dihydroxy-14α, 15α-methylen-androst-4-en-3-on,
15) 4,17β-Dihydroxy-14β, 15β-methylen-androst-4-en-3-on,
16) 4,17α-Dihydroxy-14β,15β-methylen-androst-4-en-3-on,
17) 4-Trifluormethyl-17β-Hydroxy-14α, 15α-methylen-androst-4-en-3-on,
18) 4-Trifluormethyl-17α-Hydroxy-14α, 15α-methylen-androst-4-en-3-on,
19) 4-Trifluormethyl-17β-Hydroxy-14β, 15β-methylen-androst-4-en-3-on,
20) 4-Trifluormethyl-17α-Hydroxy-14β, 15β-methylen-androst-4-en-3-on,
21) 17β-Hydroxy-14α, 15α-methyien-androsta-1,4-dien-3-on,
22) 17α-Hydroxy-14α, 15α-methylen-androsta-1,4-dien-3-on,
23) 17β-Hydroxy-14β,15β-methylen-androsta-1,4-dien-3-on,
24) 17α-Hydroxy-14β, 15β-methylen-androsta-1,4-dien-3-on,
25) 4-Chlor-17α-Hydroxy-14α, 15α-methylen-androsta-1,4-dien-3-on,
26) 4-Chlor-17β-Hydroxy-14α, 15α-methylen-androsta-1,4-dien-3-on,
27) 4-Chlor-17β-Hydroxy-14β, 15β-methylen-androsta-1,4-dien-3-on und
28) 4-Chlor-17α-Hydroxy-14β, 15β-methylen-androsta-1,4-dien-3-on.

8. Verfahren zur Herstellung der Verbindungen nach einem der Ansprüche 1 bis 7,
**dadurch gekennzeichnet,**
**daß** man in Verbindungen der allgemeinen Formel (II) 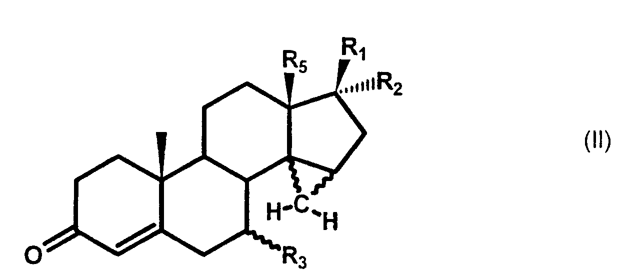 worin R₁, R₂, R₃ und R₅ die in Anspruch 1 gegebene Bedeutung besitzen und sich zwischen C-14 und C-15 eine α- oder β-Cyclopropangruppe befindet,
die 4,5-Doppelbindung mit Wasserstoffperoxid unter alkalischen Bedingungen epoxidiert, das entstandene Epoxidgemisch in einem geeignetem Lösungsmittel mit Säuren der allgemeinen Formel HR₈ behandelt, wobei R₈ ein Halogenatom, insbesondere ein Fluor-, Chlor- oder Bromatom, oder ein Pseudohalogen, insbesondere eine Rhodanid- oder Azidgruppe, sein kann, oder mit katalytischen Mengen Mineralsäure umsetzt und gegebenenfalls die erhaltenen 4-Bromverbindungen der allgemeinen Formel (I) mit 2,2-Difluor-2-(fluorsulfonyl)essigsäuremethylester in Dimethylformamid in Gegenwart von CuJ umsetzt.

9. Pharmazeutische Zusammensetzungen, die mindestens eine Verbindung der allgemeinen Formel (I) gemäß den Ansprüchen 1 bis 7, gegebenenfalls zusammen mit pharmazeutisch verträglichen Hilfs- und Trägerstoffen enthalten.

10. Verwendung der Verbindungen der allgemeinen Formel (I) gemäß den Ansprüchen 1 bis 7 zur Herstellung eines medikaments zur Fertilitätskontrolle beim Mann, der Hormonreplacement-Therapie bei Mann und Frau oder der Behandlung hormonell bedingter Erkrankungen bei Mann und Frau, wie beispielsweise Endometriose, Mammacarcinom oder Hypogonadismus.

11. Verbindungen der allgemeinen Formel (I) nach einem der Ansprüche 1 bis 7 zur Anwendung als therapeutische Wirkstoffe.

## Claims

1. Unsaturated 14,15-cyclopropano-androstanes of the general formula (I) 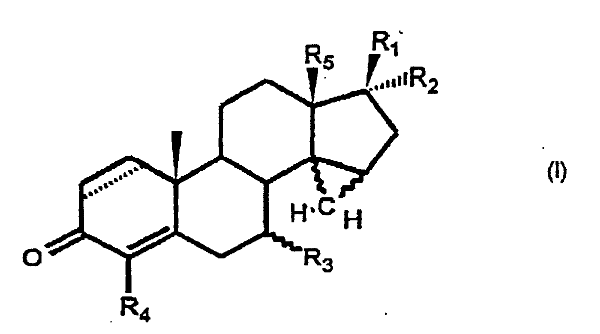 in which
R₁ represents a hydrogen atom, a hydroxy group, a C₁₋₁₀ alkyl, C₁₋₁₀ alkyloxy, C₁₋₁₅ acyloxy, C₄₋₁₅ aryloxy, C₇₋₁₅ aralkyloxy, or a C₇₋₁₅ alkylaryloxy group,
R₂ represents a hydrogen atom, a hydroxy group, C₁₋₁₀ alkyl, C₁₋₁₀ acyl, C₁₋₁₀ acyloxy, C₆₋₁₅ aryl, C₇₋₁₅ aralkyl or a C₇₋₁₅ alkylaryl group,
a -(CH₂)ₙCH₂Y group,
in which n = 0, 1 or 2 and Y represents a halogen atom, especially a fluorine, chlorine, bromine or iodine atom, a pseudohalogen, especially a cyano, azide or rhodanide group,
a -(CH₂)ₘ-CH=CH(CH₂)ₚ-R₆ group
in which m = 0, 1, 2 or 3 and p = 0, 1 or 2 and R₆ represents a hydrogen atom, a C₁₋₁₀ alkyl, C₆₋₁₅ aryl, C₇₋₁₅ aralkyl or C₇₋₁₅ alkylaryl group or a hydroxyl group, a C₁₋₁₀ alkyloxy group or a C₁₋₁₀ acyloxy group,
a -(CH₂)ₒC≡CR₇ group
in which o = 0, 1 or 2 and R₇ represents a hydrogen atom, a halogen atom, especially a fluorine, chlorine, bromine or iodine atom, a C₁₋₁₀ alkyl, C₆₋₁₅ aryl, C₇₋₁₅ aralkyl, C₇₋₁₅ alkylaryl or C₁₋₁₀ acyl group,
R₁ and R₂ together represent a keto, methylene or difluoromethylene group or, with inclusion of the C-17, form a spirooxirane or a 2,2-dimethyl-1,3-dioxolane,
there being a double bond between C-1 and C-2,
there being an α or β cyclopropane group between C-14 and C-15,
R₃ represents a hydrogen atom or an α or β C₁₋₁₀ alkyl group,
R₄ represents a halogen atom, especially a fluorine, chlorine or bromine atom or a pseudohalogen, especially a rhodanide or an azide group, or a hydroxyl or a C₁₋₃ perfluoroalkyl group and
R₅ represents a C₁₋₄ alkyl group,
whereby a C₁₋₁₀ alkyloxy group is a heterocyclic, cyclic or acyclic group, in which the alkyl rest contains 1 to 10 carbon atoms, and wherein a heterocyclic group includes also groups, which may have one or two hetero atoms in the ring, which may be selected from a nitrogen atom, an oxygen atom and a sulfur atom,
whereby the aryl rest of the C₆₋₁₅ aryl, C₇₋₁₅ alkylaryl, C₇₋₁₅ alkylaryloxy, C₇₋₁₅ aralkyl and C₇₋₁₅ aralkyloxy group may be further substituted,
with the proviso that, if there is a double bond in the 1,2 position, R₄, in addition to the meanings given above, may be a hydrogen atom, as well as their pharmaceutically tolerated salts.

2. The compounds of claim 1, in which R₁ is a hydroxy or C₁₋₁₅ acyloxy group, especially a formyloxy group, an acetyloxy group, a propionyloxy group, a butyryloxy group, an i-butyryloxy group, a heptanyloxy group or an undecanyloxy group.

3. The compounds of claim I or 2, in which R₂ represents a hydrogen atom or an alkyl group, especially a methyl or ethyl group.

4. Compounds of one of the claims 1 to 3, in which R₃ is a methyl group.

5. Compounds of one of the claims I to 4, in which R₄ is a fluorine, chlorine or bromine atom or a trifluoromethyl or a hydroxy group.

6. Compounds of one of the claims 1 to 5, in which R₅ is a methyl or ethyl group.

7. Compounds of claim 1, namely
1) 4-chloro-17β-hydroxy-14α,15α-methylene-androst-4-ene-3-one
2) 4-chloro-17α-hydroxy-14α,15α-methylene-androst-4-ene-3-one
3) 4-chloro-17β-hydroxy-14β,15β-methylene-androst-4-ene-3-one
4) 4-chloro-17α-hydroxy-14β,15β-methylene-androst-4-ene-3-one
5) 4-bromo-17β-hydroxy-14α,15α-methylene-androst-4-ene-3-one
6) 4-bromo-17α-hydroxy-14α,15α-methylene-androst-4-ene-3-one
7) 4-bromo-17β-hydroxy-14β,15β-methylene-androst-4-ene-3-one
8) 4-bromo-17α-hydroxy-14β,15β-methylene-androst-4-ene-3-one
9) 4-fluoro-17β-hydroxy-14α,15α-methylene-androst-4-ene-3-one
10) 4-fluoro-17α-hydroxy-14α,15α-methylene-androst-4-ene-3-one
11) 4-fluoro-17β-hydroxy-14β,15β-methylene-androst-4-ene-3-one
12) 4-fluoro-17α-hydroxy-14β, 15β-methylene-androst-4-ene-3-one
13) 4,17β-dihydroxy-14α,15α-methylene-androst-4-ene-3-one
14) 4,17α-dihydroxy-14α,15α-methylene-androst-4-ene-3-one
15) 4,17β-dihydroxy-14β,15β-methylene-androst-4-ene-3-one
16) 4,17α-dihydroxy-14β,15β-methylene-androst-4-ene-3-one
17) 4-trifluoromethyl-17β-hydroxy-14α,15α-methylene-androst-4-ene-3-one
18) 4-trifluoromethyl-17α-hydroxy-14α,15α-methylene-androst-4-ene-3-one
19) 4-trifluoromethyl-17β-hydroxy-14β,15β-methylene-androst-4-ene-3-one
20) 4-trifluoromethyl-17α-hydroxy-14β,15β-methylene-androst-4-ene-3-one
21) 17β-hydroxy-14α,15α-methylene-androsta-1,4-diene-3-one
22) 17α-hydroxy-14α, 15α-methylene-androsta-1,4-diene-3-one
23) 17β-hydroxy-14β,15β-methylene-androsta-1,4-diene-3-one
24) 17α-hydroxy-14β,15β-methylene-androsta-1,4-diene-3-one
25) 4-chloro-17α-hydroxy-14α,15α-methylene-androsta-1,4-diene-3-one
26) 4-chloro-17β-hydroxy-14α,15α-methylene-androsta-1,4-diene-3-one
27) 4-chloro-17β-hydroxy-14β,15β-methylene-androsta-1,4-diene-3-one and
28) 4-chloro-17α-hydroxy-14β,15β-methylene-androsta-1,4-diene-3-one

8. A method for the synthesis of compounds of one of the claims 1 to 7, wherein compounds of the general formula (II) 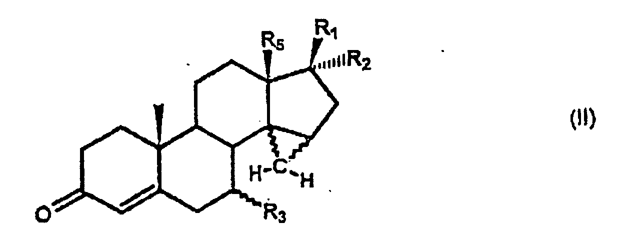 in which R₁, R₂, R₃, and R₅, which have the meanings given in claim 1, and there is an α or β cyclopropane group between C-14 and C-15, the 4,5 double bond is epoxidized under alkaline conditions with hydrogen peroxide and the resulting epoxide mixture is treated in a suitable solvent with acids of the general formula HR₈, in which R₈ may be a halogen atom, especially a fluorine, chlorine or bromine atom, or a pseudohalogen, especially a rhodanide or azide group, or reacted with catalytic amounts of a mineral acid and, optionally, the 4-bromo compounds obtained, which has the general formula (I), are reacted with methyl 2,2-difluoro-2-(fluorosulfonyl)acetate in dimethylformamide in the presence of CuI.

9. A pharmaceutical composition, which contains at least one compound of the general formula (I) of claims 1 to 7, optionally together with pharmaceutically tolerated inactive materials and vehicles.

10. The use of compounds of the general formula (I) of claims 1 to 7 for the production of a medicament for controlling the fertility in men, for hormone replacement therapy in men and women or for the treatment of hormone-induced diseases in men and women, such as endometriosis, breast cancer or hypogonadism.

11. Compounds of the general formula (I) of claims 1 to 7 for use as active therapeutic ingredients.

## Revendications

1. 14,15-cyclopropano-androstanes insaturés de formule générale (I) 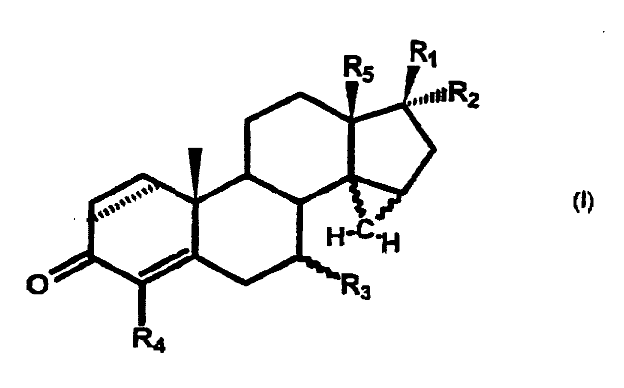 dans laquelle
R₁ est un atome d'hydrogène, un groupe hydroxy, un groupe alkyle en C₁₋₁₀, un groupe alkyloxy en C₁₋₁₀, un groupe acyloxy en C₁₋₁₅, un groupe aryloxy en C₄₋₁₅, un groupe aralkyloxy en C₇₋₁₅ ou un groupe. alkylaryloxy en C₇₋₁₅,
R₂ représente un atome d'hydrogène, un groupe hydroxy, un groupe alkyle en C₁₋₁₀, un groupe acyle en C₁₋₁₀, un groupe acyloxy en C₁₋₁₀, un groupe aryle en C₆₋₁₅, un groupe aralkyle en C₇₋₁₅ ou un groupe alkylaryle en C₇₋₁₅,
un radical -(CH₂)ₙCH₂Y
dans lequel n = 0,1 ou 2 et Y représente un atome d'halogène, notamment un atome de fluor, de chlore, de brome ou d'iode, ou un pseudohalogène, notamment un groupe cyano, azide ou thiocyanate,
un radical -(CH₂)ₘ-CH=CH(CH₂)ₚ-R₆
dans lequel m = 0, 1, 2 ou 3 et p = 0, 1 ou 2 et R₆ représente un atome d'hydrogène, un radical alkyle en C₁₋₁₀, un radical aryle en C₆₋₁₅, un radical aralkyle en C₇₋₁₅, un radical alkylaryle en C₇₋₁₅, ou un groupe hydroxyle, un groupe alkyloxy en C₁₋₁₀, ou un groupe acyloxy en C₁₋₁₀,
un radical -(CH₂)ₒC≡CR₇
dans lequel o = 0, 1 ou 2 et R₇ représente un atome d'hydrogène, un atome d'halogène, notamment un atome de fluor, de chlore, de brome ou d'iode, un radical alkyle en C₁₋₁₀, un radical aryle en C₆₋₁₅, un radical aralkyle en C₇₋₁₅, un radical alkylaryle en C₇₋₁₅, ou un radical acyle en C₁₋₁₀,
R₁ et R₂ représentent ensemble un groupe céto, méthylène ou difluorométhylène ou forment par inclusion du C-17 un spirooxane ou un 2,2-diméthyl-1,3-dioxolane,
entre C-1 et C-2, il peut se trouver une double liaison,
entre C-14 et C-15, il se trouve un groupe α- ou β-cyclopropane,
R₃ représente un atome d'hydrogène ou un groupe alkyle en C₁₋₁₀ en position α ou β,
R₄ représente un atome d'halogène, notamment un atome de fluor, de chlore, ou de brome, ou un pseudohalogène, notamment un groupe thiocyanate ou azide, ou un groupe hydroxyle ou perfluoroalkyle en C₁₋₃,
R₅ représente un groupe alkyle en C₁₋₄,
dans laquelle le groupe alkyloxy en C₁₋₁₀ représente un groupe hétérocyclique, cyclique ou acyclique, dont le radical alkyle comporte 1 à 10 atomes de carbone, le groupe hétérocyclique pouvant comporter 1 à 2 hétéroatomes dans le noyau, choisis parmi N, O et S,
et dans laquelle les résidus aryle du groupe aryle en C₆₋₁₅, alkylaryle en C₇₋₁₅, alkylaryloxy en C₇₋₁₅, aralkyle en C₇₋₁₅ et aralkyloxy en C₇₋₁₅ peuvent être substitués en plus,
sous réserve que s'il se trouve une double liaison en position 1,2, R₄ peut représenter, en plus des désignations précédentes, un atome d'hydrogène, ainsi que leurs sels pharmaceutiquement compatibles.

2. Composés selon la revendication 1, dans lesquels R₁ représente un groupe hydroxy ou acyloxy en C₁₋₁₅, notamment un groupe formyloxy, acétyloxy, propionyloxy, butyrytoxy, iso-butyryloxy, heptanyloxy ou undécanyloxy.

3. Composés selon la revendication 1 ou 2, dans lesquels R₂ représente un atome d'hydrogène ou un groupe alkyle, notamment un groupe méthyle ou éthyle.

4. Composés selon une des revendications 1 à 3, dans lesquels R₃ est un groupe méthyle.

5. Composés selon une des revendications 1 à 4, dans lesquels R₄ représente un atome de fluor, de chlore ou de brome, ou un groupe trifluorométhyle ou hydroxy.

6. Composés selon une des revendications 1 à 5, dans lesquels R₅ représente un groupe méthyle ou éthyle.

7. Composés selon la revendication 1, à savoir :
1) 4-chloro-17β-hydroxy-14α, 15α-méthylène-androst-4-én-3-one,
2) 4-chloro-17α-hydroxy-14α, 15α-méthylène-androst-4-én-3-one,
3) 4-chloro-17β-hydroxy-14β, 15β-méthylène-androst-4-én-3-one,
4) 4-chloro-17α-hydroxy-14β, 15β-méthylène-androst-4-én-3-one,
5) 4-bromo-17β-hydroxy-14α, 15α-méthylène-androst-4-én-3-one,
6) 4-bromo-17α-hydroxy-14α, 15α-méthylène-androst-4-én-3-one,
7) 4-bromo-17β-hydroxy-14β, 15β-méthylène-androst-4-én-3-one,
8) 4-bromo-17α-hydroxy-14β, 15β-méthylène-androst-4-én-3-one,
9) 4-fluoro-17β-hydroxy-14α, 15α-méthylène-androst-4-én-3-one,
10) 4-fluoro-17α-hydroxy-14α, 15α-méthylène-androst-4-én-3-one,
11) 4-fluoro-17β-hydroxy-14β, 15β-méthylène-androst-4-én-3-one,
12) 4-fluoro-17α-hydroxy-14β, 15β-méthylène-androst-4-én-3-one,
13) 4,17β-dihydroxy-14α,15α-méthylène-androst-4-én-3-one,
14) 4,17α-dihydroxy-14α, 15α-méthylène-androst-4-én-3-one,
15) 4,17β-dihydroxy-14β, 15β-méthylène-androst-4-én-3-one,
16) 4,17α-dihydroxy-14β,15β-méthylène-androst-4-én-3-one,
17) 4-trifluorométhyl-17β-hydroxy-14α, 15α-méthylène-androst-4-én-3-one,
18) 4-trifluorométhyl-17α-hydroxy-14α, 15α-méthylène-androst-4-én-3-one,
19) 4-trifluorométhyl-17β-hydroxy-14β, 15β-méthylène-androst-4-én-3-one.
20) 4-trifluorométhyl-17α-hydroxy-14β, 15β-méthylène-androst-4-én-3-one,
21) 17β-hydroxy-14α,15α-méthylène-androsta-1,4-dién-3-one,
22) 17α-hydroxy-14α, 15α-méthylène-androsta-1,4-dién-3-one,
23) 17β-hydroxy-14β, 15β-méthytène-androsta-1,4-dién-3-one,
24) 17α-hydroxy-14β, 15β-méthylène-androsta-1,4-dién-3-one,
25) 4-chloro-17α-hydroxy-14α, 15α-méthylène-androsta-1,4-dién-3-one,
26) 4-chloro-17β-hydroxy-14α,15α-méthylène-androsta-1,4-dién-3-one,
27) 4-chloro-17β-hydroxy-14β, 15β-méthylène-androsta-1,4-dién-3-one, et
28) 4-chloro-17α-hydroxy-14β,15β-méthylène-androsta-1,4-dién-3-one.

8. Procédé de préparation des composés selon une des revendications 1 à 7, **caractérisé en ce**
**que**, dans des composés de formule générale (II) 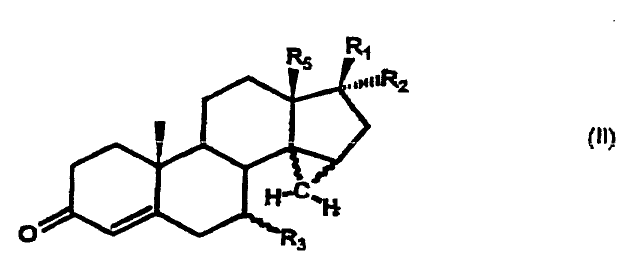 dans laquelle R₁, R₂, R₃ et R₅ ont la signification indiquée dans la revendication 1 et un groupe α-ou β-cyclopropane se trouve entre C-14 et C-15,
on époxyde la double liaison 4,5 avec du peroxyde d'hydrogène dans des conditions alcalines, le mélange époxyde obtenu est traité dans un solvant approprié avec des acides de formule générale HR₈, dans laquelle R₈ peut être un atome d'halogène, notamment un atome de fluor, de chlore ou de brome, ou un pseudohalogène, notamment un groupe thiocyanate ou azide, ou il est transformé avec des quantités catalytiques d'un acide minéral, et on transforme éventuellement les composés 4-bromo obtenus de formule générale (1) avec du méthylester d'acide 2,2-difluoro-2-(fluorosulfonyl)acétique dans du diméthylformamide en présence de Cul.

9. Compositions pharmaceutiques, qui contiennent au moins un composé de formule générale (I) selon les revendications 1 à 7, éventuellement conjointement avec des agents auxiliaires ou des agents de support pharmaceutiquement compatibles.

10. Utilisation des composés de formule générale (1) selon les revendications 1 à 7 pour préparer un médicament destiné au contrôle de la fertilité chez l'homme, à l'hormonothérapie de substitution chez l'homme et la femme, ou au traitement des maladies d'origine hormonale chez l'homme et la femme, comme par exemple l'endométriose, le cancer du sein ou l'hypogonadisme.

11. Composés de formule générale (I) selon une des revendications 1 à 7 destinés à une application en tant que principes actifs thérapeutiques.
